# EUROPEAN PATENT APPLICATION

(11) **EP 2 444 001 A1**
(43) Date of publication of application: **25.04.2012**
(21) Application number: 11176396.7
(22) Date of filing: 03.08.2011
(51) Int. Cl.: A61B 8/08, G01S 15/89

(54) **Providing an ultrasound spatial compound image based on a phased array probe in an ultrasound system**

(30) Priority: 20.10.2010 KR 20100102629
(71) Applicant: Samsung Medison Co., Ltd., Nam-myun Hongchun-gun Kangwon do 250-875 (KR)
(72) Inventor: Lee, Kwang Ju, 135-851 Seoul (KR)
(74) Representative: Schmid, Wolfgang

(57) **Abstract**

Embodiments for providing an ultrasound spatial compound image are disclosed. In one embodiment, by way of non-limiting example, an ultrasound system comprises: an ultrasound data acquisition unit configured to acquire ultrasound data based on a phased array probe having a plurality of elements; and a processing unit in communication with the ultrasound data acquisition unit, the processing unit being configured to set a virtual common point corresponding to predetermined scan-lines, move the virtual common point in a longitudinal direction of the elements to set a plurality of scan-lines, form a plurality of ultrasound images based on the ultrasound data, and perform spatial compounding upon the ultrasound images to form an ultrasound spatial compound image, wherein the ultrasound data acquisition unit is configured to acquire the ultrasound data based on the plurality of scan-lines corresponding to each of the ultrasound images.

## Description

### CROSS-REFERENCE TO RELATED APPLICATION

The present application claims priority from Korean Patent Application No. 10-2010-0102629 filed on October 20, 2010.

### TECHNICAL FIELD

The present disclosure generally relates to ultrasound systems, and more particularly to providing an ultrasound spatial compound image based on a phased array probe in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two-dimensional or three-dimensional ultrasound images of internal features of a target object (e.g., human organs).

The ultrasound system may transmit and receive ultrasound signals to and from a living body to thereby form a 2D (two-dimensional) ultrasound image or a 3D (three-dimensional) ultrasound image. Various techniques have been studied to enhance resolution of the ultrasound image. Spatial compounding is known as one of such techniques.

Spatial compounding is an imaging technique for forming a compound image by combining ultrasound images. That is, the ultrasound system forms a plurality of ultrasound images and performs the spatial compounding upon the ultrasound images to form an ultrasound spatial compound image.

The ultrasound system sets a virtual common point, at which scan-lines intersect by extending the scan-lines to back of elements of a convex probe, and moves the virtual common point to particular positions to thereby set a plurality of scan-lines corresponding to each of the ultrasound images. However, there is a problem since the ultrasound spatial compound image cannot be formed by using a phased array probe.

### SUMMARY

Embodiments for providing an ultrasound spatial compound image in an ultrasound system are disclosed herein. In one embodiment, by way of non-limiting example, an ultrasound system comprises: an ultrasound data acquisition unit configured to acquire ultrasound data based on a phased array probe having a plurality of elements; and a processing unit in communication with the ultrasound data acquisition unit, the processing unit being configured to set a virtual common point corresponding to predetermined scan-lines, move the virtual common point in a longitudinal direction of the elements to set a plurality of scan-lines, form a plurality of ultrasound images based on the ultrasound data, and perform spatial compounding upon the ultrasound images to form an ultrasound spatial compound image, wherein the ultrasound data acquisition unit is configured to acquire the ultrasound data based on the plurality of scan-lines corresponding to each of the ultrasound images.

In another embodiment, there is a method of providing an ultrasound spatial compound image based on a phased array probe having a plurality of elements, comprising: a) setting a virtual common point corresponding to predetermined scan-lines; b) moving the virtual common point in a longitudinal direction of the elements to set a plurality of scan-lines; c) acquiring ultrasound data by transmitting and receiving ultrasound signals based on the plurality of scan-lines; d) forming a plurality of ultrasound images based on the ultrasound data; and e) performing spatial compounding upon the ultrasound images to form an ultrasound spatial compound image.

This summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG 1: is a block diagram showing an illustrative embodiment of an ultrasound system.
- FIG 2: is a block diagram showing an illustrative embodiment of an ultrasound data acquisition unit.
- FIG 3: is a flow chart showing a process of forming an ultrasound spatial compound image.
- FIG 4: is a schematic diagram showing an example of a plurality of scan-lines and a virtual common point.
- FIG 5: is a schematic diagram showing an example of ultrasound images and subvirtual common points.
- FIG 6: is a schematic diagram showing another example of the ultrasound images and the sub-virtual common points.

### DETAILED DESCRIPTION

A detailed description may be provided with reference to the accompanying drawings. One of ordinary skill in the art may realize that the following description is illustrative only and is not in any way limiting.

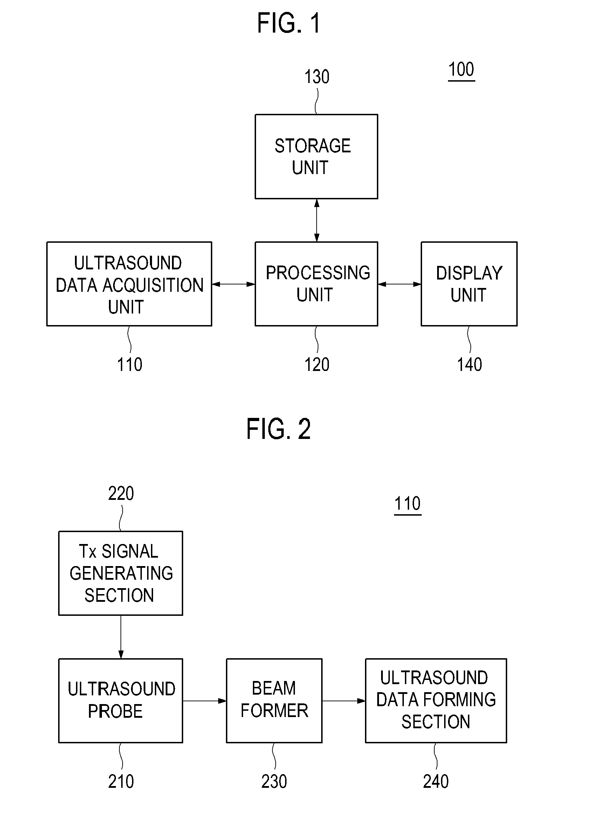

Referring to FIG 1, an ultrasound system 100 in accordance with an illustrative embodiment is shown. As depicted therein, the ultrasound system 100 includes an ultrasound data acquisition unit 110. The ultrasound data acquisition unit 110 is configured to transmit and receive ultrasound signals to and from a living body to acquire ultrasound data. The living body includes a plurality of target objects (e.g., blood vessels, a heart, etc.).

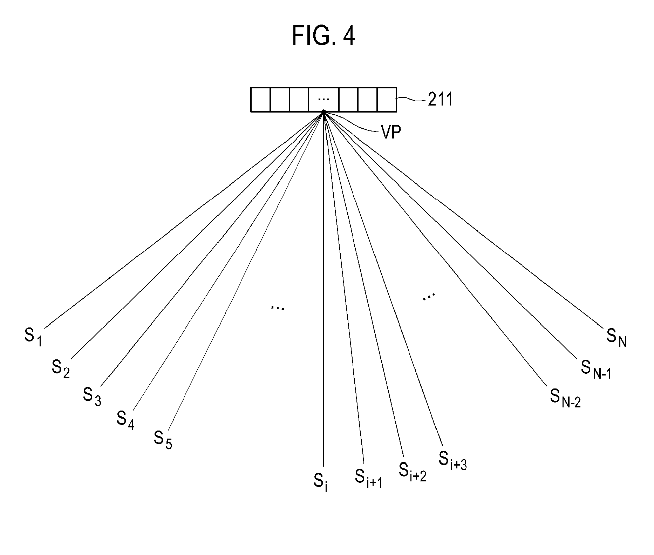

FIG 2 is a block diagram showing an illustrative embodiment of the ultrasound data acquisition unit 110. Referring to FIG 2, the ultrasound data acquisition unit 110 includes an ultrasound probe 210. The ultrasound probe 210 includes a plurality of elements 211 (*see* FIG 4) for reciprocally converting between ultrasound signals and electrical signals. The ultrasound probe 210 is configured to transmit ultrasound signals to the living body along each of a plurality of scan-lines. The ultrasound probe 210 is further configured to receive ultrasound signals (i.e., ultrasound echo signals) from the living body to output received signals. The received signals are analog signals. The ultrasound probe 210 includes a phased array probe.

The ultrasound data acquisition unit 110 further includes a transmit (Tx) signal generating section 220. The Tx signal generating section 220 is configured to control the transmission of the ultrasound signals. The Tx signal generating section 220 is further configured to generate electrical signals ("Tx signals") for obtaining at least one ultrasound image in consideration of the elements and focal points. The ultrasound image includes a brightness mode image. However, it should be noted herein that the ultrasound image may not be limited thereto.

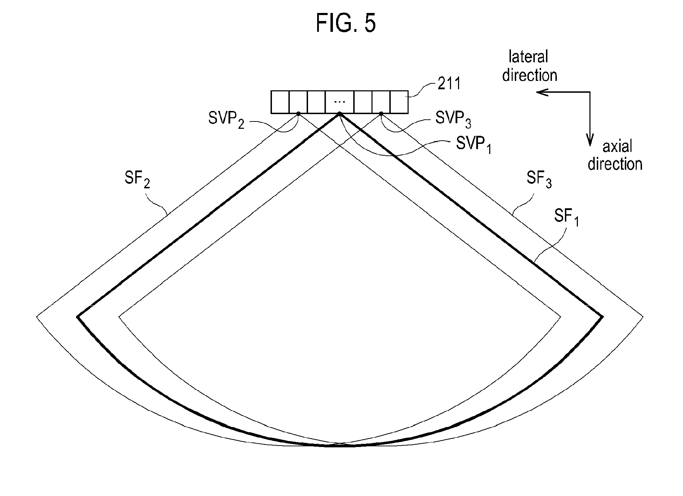

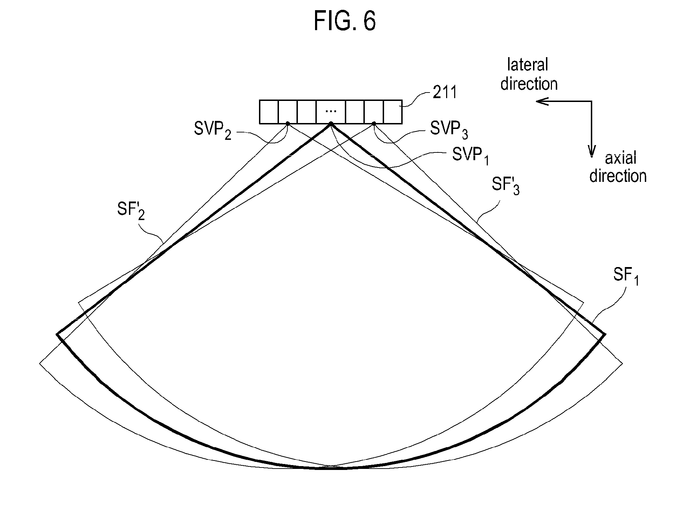

In one embodiment, the Tx signal generating section 220 is configured to generate first Tx signals for obtaining a first ultrasound image SF₁ as shown in FIG 5 or FIG 6. Thus, the ultrasound probe 210 is configured to convert the first Tx signals provided from the Tx signal generating section 220 into the ultrasound signals, transmit the ultrasound signals to the living body and receive the ultrasound echo signals from the living body to thereby output first received signals. The Tx signal generating section 220 is further configured to generate second Tx signals for obtaining a second ultrasound image SF₂ as shown in FIG 5 or FIG 6. As such, the ultrasound probe 210 is configured to convert the second Tx signals provided from the Tx signal generating section 220 into the ultrasound signals, transmit the ultrasound signals to the living body and receive the ultrasound echo signals from the living body to thereby output second received signals. The Tx signal generating section 220 is further configured to generate third TX signals for obtaining a third ultrasound image SF₃ as shown in FIG 5 or FIG 6. Accordingly, the ultrasound probe 210 is configured to convert the third Tx signals provided from the Tx signal generating section 220 into the ultrasound signals, transmit the ultrasound signals to the living body and receive the ultrasound echo signals from the living body to thereby output third received signals.

The number of ultrasound images is determined depending on the number of the ultrasound images, which are needed to form an ultrasound spatial compound image. The ultrasound data acquisition unit 110 further includes a beam former 230. The beam former 230 is configured to convert the received signals provided from the ultrasound probe 210 into digital signals. The beam former 230 is further configured to apply delays to the digital signals in consideration of the elements and the focal points to thereby output digital receive-focused signals.

In one embodiment, the beam former 230 is configured to convert the first received signals provided from the ultrasound probe 210 into first digital signals. The beam former 230 is further configured to apply delays to the first digital signals in consideration of the elements and the focal points to thereby output first digital receive-focused signals. The beam former 230 is also configured to convert the second received signals provided from the ultrasound probe 210 into second digital signals. The beam former 230 is additionally configured to apply delays to the second digital signals in consideration of the elements and the focal points to thereby output second digital receive-focused signals. The beam former 230 is further configured to convert the third received signals provided from the ultrasound probe 210 into third digital signals. The beam former 230 is additionally configured to apply delays to the third digital signals in consideration of the elements and the focal points to thereby output third digital receive-focused signals.

The ultrasound data acquisition unit 110 further includes an ultrasound data forming section 240. The ultrasound data forming section 240 is configured to form ultrasound data corresponding to the ultrasound image based on the digital receive-focused signals. The ultrasound data include radio frequency data. However, it should be noted herein that the ultrasound data may not be limited thereto. The ultrasound data forming section 240 is also configured to perform signal processing (e.g., gain control, etc) upon the digital receive-focused signals.

In one embodiment, the ultrasound data forming section 240 is configured to form first ultrasound data corresponding to the first ultrasound image SF₁ based on the first digital receive-focused signals provided from the beam former 230. The ultrasound data forming section 240 is further configured to form second ultrasound data corresponding to the second ultrasound image SF₂ based on the second digital receive-focused signals provided from the beam former 230. The ultrasound data forming section 240 is also configured to form third ultrasound data corresponding to the third ultrasound image SF₃ based on the third digital receive-focused signals provided from the beam former 230.

Referring back to FIG 1, the ultrasound system 100 further includes a processing unit 120 in communication with the ultrasound data acquisition unit 110. The processing unit 120 includes a central processing unit, a microprocessor or a graphic processing unit. However, it should be noted herein that the processing unit 120 may not be limited thereto.

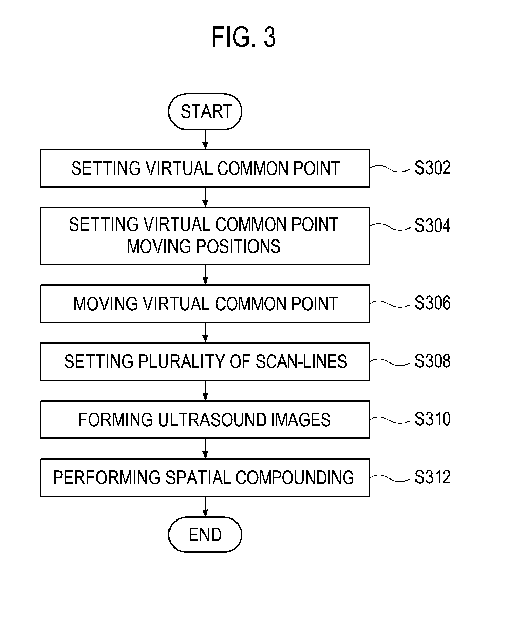

FIG 3 is a flow chart showing a process of forming an ultrasound spatial compound image. The processing unit 120 is configured to set a virtual common point VP corresponding to scan-lines S₁ to S_{N} in consideration of positions of the elements 211 as shown in FIG 4, at step S302 in FIG 3. The virtual common point VP is a point, at which the scan-lines S₁ to S_{N} intersect.

The processing unit 120 is configured to set virtual common point moving positions for moving the virtual common point VP in a lateral direction based on the virtual common point VP, at step S304 in FIG 3. The lateral direction is a longitudinal direction of the elements 211 as shown in FIG 5. In FIG 5, the axial direction is a Tx direction of the ultrasound signals.

The processing unit 120 is configured to move the virtual common point VP to the virtual common point moving positions to set sub-virtual common points corresponding to the ultrasound images, at step S306 in FIG 3.

In one embodiment, the processing unit 120 is configured to set a first virtual common point moving position corresponding to the first ultrasound image SF₁ based on the virtual common point VP. That is, the processing unit 120 sets a position of the virtual common point VP as the first virtual common point moving position. The processing unit 120 is further configured to set a first sub-virtual common point SVP₁ corresponding to the first ultrasound image SF₁ based on the first virtual common point moving position, as shown in FIG 5. The processing unit 120 is also configured to set a second virtual common point moving position for moving the virtual common point VP in the lateral direction by a predetermined distance based on the virtual common point VP. The processing unit 120 is further configured to move the virtual common point VP to the second virtual common point moving position to set a second virtual common point SVP₂ corresponding to the second ultrasound image SF₂, as shown in FIG 5. The processing unit 120 is also configured to set a third virtual common point moving position for moving the virtual common point VP in the lateral direction by a predetermined distance based on the virtual common point VP. The processing unit 120 is further configured to move the virtual common point VP to the third virtual common point moving position to set a third virtual common point SVP₃ corresponding to the third ultrasound image SF₃, as shown in FIG 5. A distance between the virtual common point VP and the second sub-virtual common point SVP₂ and a distance between the virtual common point VP and the third sub-virtual common point SVP₃ are either same or different.

The processing unit 120 is configured to set a plurality of scan-lines corresponding to each of the ultrasound images based on the virtual common point and the sub-virtual common points, at step S308 in FIG 3. Thus, the ultrasound data acquisition unit 110 is configured to transmit the ultrasound signals to the living body along the set scan-lines and receive the ultrasound echo signals from the living body to acquire the ultrasound data corresponding to each of the ultrasound images.

In one embodiment, the processing unit 120 is configured to set a reference ultrasound image from the ultrasound images SF₁ to SF_{3.} The processing unit 120 sets the first ultrasound image SF₁, which does not move the virtual common point VP, as the reference ultrasound image. The processing unit 120 is further configured to set a plurality of scan-lines corresponding to the reference ultrasound image (i.e., first ultrasound image SF₁). That is, the processing unit 120 sets the scan-lines S₁ to S_{N} shown in FIG 4 as the plurality of scan-lines corresponding to the reference ultrasound image. The processing unit 120 is further configured to move the first sub-virtual common point SVP₁ (i.e., virtual common point VP) to the second virtual common point SVP₂ to set a plurality of scan-lines (i.e., steering angles corresponding to the scan-lines) corresponding to the second ultrasound images SF₂. That is, the processing unit 120 sets the plurality of scan-lines corresponding to the reference ultrasound image as the plurality of scan-lines corresponding to the second ultrasound image SF₂. The processing unit 120 is further configured to move the first sub-virtual common point SVP₁ (i.e., virtual common point VP) to the third sub-virtual common point SVP₃ to set a plurality of scan-lines (i.e., steering angles corresponding to the scan-lines) corresponding to the third ultrasound image SF₃. That is, the processing unit 120 sets the plurality of scan-lines corresponding to the reference ultrasound image as the plurality of scan-lines corresponding to the third ultrasound image SF_{3.}

Optionally, the processing unit 120 is configured to rotate the second ultrasound image SF₂ toward the first ultrasound image SF₁ at a predetermined rotating angle on a basis of the second sub-virtual common point SVP₂. The processing unit 120 is further configured to reset a plurality of scan-lines corresponding to the rotated second ultrasound image SF₂ based on the predetermined rotating angle, as shown in FIG 6. The processing unit 120 is also configured to rotate the third ultrasound image SF₃ toward the first ultrasound image SF₁ at a predetermined rotating angle on a basis of the third sub-virtual common point SVP₃. The processing unit 120 is further configured to reset a plurality of scan-lines corresponding to the rotated third ultrasound image SF₃ based on the predetermined rotating angle, as shown in FIG 6.

The processing unit 120 is configured to form the ultrasound images based on the ultrasound data provided from the ultrasound data acquisition unit 110, at step S310 in FIG 3.

In one embodiment, the processing unit 120 is configured to form the first ultrasound image SF₁ based on the first ultrasound data provided from the ultrasound data acquisition unit 110. The processing unit 120 is further configured to form the second ultrasound image SF₂ based on the second ultrasound data provided from the ultrasound data acquisition unit 110. The processing unit 120 is also configured to form the third ultrasound image SF₃ based on the third ultrasound data provided from the ultrasound data acquisition unit 110.

The processing unit 120 is configured to perform spatial compounding upon the ultrasound images to form an ultrasound spatial compound image, at step S312 in FIG 3. The methods of performing the spatial compounding are well known in the art. Thus, they have not been described in detail so as not to unnecessarily obscure the present invention. For example, the processing unit 120 performs the spatial compounding by calculating a mean brightness value corresponding to each of the pixels of the ultrasound images.

Referring back to FIG 1, the ultrasound system 100 further includes a storage unit 130. The storage unit 130 stores the ultrasound data acquired by the ultrasound data acquisition unit 110. Also, the storage unit 130 stores the ultrasound images formed by the processing unit 120.

The ultrasound system 100 further includes a display unit 140. The display unit 140 is configured to display the ultrasound spatial compound image. The display unit 140 is also configured to display the ultrasound images.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to acquire ultrasound data based on a phased array probe having a plurality of elements; and
a processing unit in communication with the ultrasound data acquisition unit,
the processing unit being configured to set a virtual common point corresponding to predetermined scan-lines, move the virtual common point in a longitudinal direction of the elements to set a plurality of scan-lines, form a plurality of ultrasound images based on the ultrasound data, and perform spatial compounding upon the ultrasound images to form an ultrasound spatial compound image,
wherein the ultrasound data acquisition unit is configured to acquire the ultrasound data based on the plurality of scan-lines corresponding to each of the ultrasound images.

2. The ultrasound system of Claim 1, wherein the processing unit is configured to:
set virtual common point moving positions for moving the virtual common point in the longitudinal direction on a basis of the virtual common point;
move the virtual common point to the virtual common point moving positions to set sub-virtual common points corresponding to the ultrasound images; and
set the plurality of scan-lines corresponding to each of the ultrasound images based on the virtual common point and the sub-virtual common points.

3. The ultrasound system of Claim 2, wherein the processing unit is configured to;
set a reference ultrasound image from the ultrasound images;
set a position of the virtual common point as the virtual common point moving position corresponding to the reference ultrasound image; and
set remaining virtual common point moving positions corresponding to remaining ultrasound images based on the virtual common point moving position corresponding to the reference ultrasound image.

4. The ultrasound system of Claim 3, wherein the processing unit is configured to:
set the predetermined scan-lines as the plurality of scan-lines corresponding to reference ultrasound image; and
set the plurality of scan-lines corresponding to each of the remaining ultrasound images based on the plurality of the scan-lines corresponding to the reference ultrasound image.

5. The ultrasound system of Claim 3, wherein the processing unit is further configured to:
rotate the remaining ultrasound images toward the reference ultrasound image at predetermined rotating angles based on the sub-virtual common points corresponding to the remaining ultrasound images; and
reset the plurality of scan-lines corresponding to the remaining ultrasound images.

6. A method of providing an ultrasound spatial compound image based on a phased array probe having a plurality of elements, comprising:
a) setting a virtual common point corresponding to predetermined scan-lines;
b) moving the virtual common point in a longitudinal direction of the elements to set a plurality of scan-lines;
c) acquiring ultrasound data by transmitting and receiving ultrasound signals based on the plurality of scan-lines;
d) forming a plurality of ultrasound images based on the ultrasound data; and
e) performing spatial compounding upon the ultrasound images to form an ultrasound spatial compound image.

7. The method of Claim 6, wherein the step b) comprises:
b1) setting virtual common point moving positions for moving the virtual common point in the longitudinal direction on a basis of the virtual common point;
b2) moving the virtual common point to the virtual common point moving positions to set sub-virtual common points corresponding to the ultrasound images; and
b3) setting the plurality of scan-lines corresponding to each of the ultrasound images based on the virtual common point and the sub-virtual common points.

8. The method of Claim 7, wherein the step b1) comprises:
setting a reference ultrasound image from the ultrasound images;
setting a position of the virtual common point as the virtual common point moving position corresponding to the reference ultrasound image; and setting remaining virtual common point moving positions corresponding to remaining ultrasound images based on the virtual common point moving position corresponding to the reference ultrasound image.

9. The method of Claim 8, wherein the step b3) comprises:
setting the predetermined scan-lines as the plurality of scan-lines corresponding to reference ultrasound image; and
setting the plurality of scan-lines corresponding to each of the remaining ultrasound images based on the plurality of the scan-lines corresponding to the reference ultrasound image.

10. The method Claim 7, wherein the step b) further comprises:
rotating the remaining ultrasound images toward the reference ultrasound image at predetermined rotating angles based on the sub-virtual common points corresponding to the remaining ultrasound images; and
resetting the plurality of scan-lines corresponding to the remaining ultrasound images.
